(19) Europäisches Patentamt
European Patent Office
Office européen des brevets

(11) Publication number: **0 079 555**
**B1**

(12) # EUROPEAN PATENT SPECIFICATION

(45) Date of publication of patent specification: **10.04.85**

(51) Int. Cl.⁴: **C 07 C 43/17** // C07C59/135, C08F216/14, C07C41/24, C07C51/58

(21) Application number: **82110284.5**

(22) Date of filing: **08.11.82**

(54) **Perfluoro(2-bromoethylvinyl ether).**

(30) Priority: **18.11.81 JP 183720/81**

(43) Date of publication of application: **25.05.83 Bulletin 83/21**

(45) Publication of the grant of the patent: **10.04.85 Bulletin 85/15**

(84) Designated Contracting States: **DE FR GB IT**

(56) References cited: **EP-A-0 041 739**

(73) Proprietor: **ASAHI GLASS COMPANY LTD.**
**No. 1-2, Marunouchi 2-chome**
**Chiyoda-ku, Tokyo (JP)**

(72) Inventor: **Kojima, Gen**
**2-11-6, Tuskushino**
**Machida-shi Tokyo (JP)**
Inventor: **Kodama, Shunichi**
**2-59-1, Tsurugamine Asahi-ku**
**Yokohama-shi Kanagawa-ken (JP)**
Inventor: **Yamabe, Masaaki**
**2-3-13, Minami-tsukushino**
**Machida-shi Tokyo (JP)**
Inventor: **Kaneko, Isamu**
**2-15-10, Rinkan**
**Yamato-shi Kanagawa-ken (JP)**

(74) Representative: **Wächtershäuser, Günter, Dr.**
**Tal 29**
**D-8000 München 2 (DE)**

EP 0 079 555 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to perfluoro(2 - bromoethylvinyl ether).

Perfluoro(2 - bromoethylvinyl ether) represented by the formula $CF_2{=}CFO(CF_2)_2Br$ is a novel compound which has never been disclosed in a literature.

The compound of the present invention is useful as an intermediate for various fluorine-containing compounds and particularly useful as a comonomer which gives a reactive site to a fluorine-containing polymer.

As bromine-containing unsaturated compounds useful as comonomers capable of giving reactive sites to fluorine-containing polymers, there have hitherto been known olefins such as bromotrifluoroethylene ($CF_2{=}CFBr$), 4 - bromo - 3,3,4,4 - tetrafluorobutene - 1 ($CH_2{=}CHCF_2CF_2Br$), vinylbromide ($CH_2{=}CHBr$), 1 - bromo - 2,2 - difluoroethylene ($CF_2{=}CHBr$), 3 - bromoperfluoropropene ($CF_2{=}CFCF_2Br$), 4 - bromo - 1,1,2 - trifluorobutene ($CF_2{=}CFCH_2CHBr$), 4 - bromo - 1,1,3,3,4,4 - hexafluorobutene ($CF_2{=}CHCF_2CF_2Br$), 4 - bromo - 3 - chloro - 1,1,3,4,4 - pentafluorobutene ($CF_2{=}CHCFClCF_2Br$), 6 - bromo - 5,5,6,6 - tetrafluorohexene ($CH_2{=}CHCH_2CH_2CF_2CF_2Br$), 4 - bromoperfluorobutene-1 ($CF_2{=}CFCF_2CF_2Br$) and 3 - bromo - 3,3 - difluoropropene ($CH_2{=}CHCF_2Br$) (see Apotheker et al, U.S. Patent No. 4,035,565).

However, a perfluorovinyloxy group-containing unsaturated bromine compounds having an ether linkage like the compound of the present invention is novel.

The compound of the present invention has various advantages as a comonomer capable of giving a reactive site to fluorine-containing polymers over the known fluorine-containing olefins, for instance, as follows:

(1) It has a monomer reactivity as a perfluorovinyl ether, and accordingly it can readily be copolymerized with various fluoroolefins and it does not bring about a substantial reduction of the polymerization rate and of the molecular weight of the resultant copolymer;

(2) It will be taken in the main chain of the copolymer in a form of a perfluorovinyl group, and accordingly, it contributes to an improvement of the thermal stability and the chemical stability of the main chain;

(3) The side chain is a perfluoro ether group, and accordingly flexibility will be imparted to the copolymer and at the same time, the heat resistance, chemical resistance, solvent resistance and oil resistance of the copolymer will be improved.

The compound of the present invention is applicable as a comonomer capable of giving a reactive site not only to fluorine-containing polymers but also to various other polymers. Its application to fluorine-containing polymers is, however, preferred since the above mentioned various advantages can thereby be attained. As the fluorine-containing polymers to which the compound of the present invention can advantageously be applied, there may be mentioned a tetrafluoroethylene-propylene copolymer, a vinylidene fluoride-hexafluoropropene copolymer, a vinylidene fluoride-perfluorovinyl ether copolymer, a tetrafluoroethylene-perfluorovinyl ether copolymer, a polytetrafluoroethylene, a polychlorotrifluoroethylene, a polyvinylidene fluoride, an ethylene-tetrafluoroethylene copolymer, an ethylene-chlorotrifluoroethylene copolymer and a tetrafluoroethylene-hexafluoropropene copolymer. It can advantageously be applied for the formation of a cure site for elastomeric polymers. However, it is also applicable to resinous polymers.

When the compound of the present invention is to be used for the above mentioned purpose, it is copolymerized usually in an amount of from 0.05 to 20 molar %, preferably from 0.1 to 10 molar % at the time of forming the above mentioned polymers, whereby copolymers having a reactive site can be produced. The copolymers thus obtained can readily be converted to useful cross-linked products e.g. by a method of subjecting it to peroxide curing in the presence of a polyfunctional compound such as triallylisocyanurate.

As a typical example of the application of the compound of the present invention as an intermediate, there may be mentioned a production of a divinyl compound represented by the formula of $CF_2{=}CFO(CF_2)_4OCF{=}CF_2$ by a debromination coupling reaction. The divinyl compound is useful as a comonomer component for the formation of a partially gelled polymer or a cross-linked polymer.

The compound of the present invention may be prepared by various methods. It is, however, advantageous to employ a method which comprises reacting bromodifluoroacetyl fluoride and hexafluoropropeneoxide in an organic solvent containing no active hydrogen such as diglyme in the presence of a fluorine anion source such as an alkali metal fluoride to form perfluoro(5 - bromo - 3 - oxa - 2 - methyl - pentanoylfluoride) $BrCF_2CF_2OCF(CF_3)COF$, which is then directly, or after once hydrolysed and neutralized with e.g. potassium hydroxide, subjected to pyrolysis. The starting material bromodifluoroacetyl fluoride can be obtained in good yield e.g. by a method which comprises reacting tetrafluoroethylene with a reaction reagent obtained by mixing bromine with sulfur trioxide and then allowing the resulting intermediate containing a $BrCF_2CF_2OSO_2$-group to decompose, or a method which comprises oxidizing 1,2 - dibromo - 2 - chlorotrifluoroethylene with fuming sulfuric acid.

In the practical operation of the above method, various combinations of reaction conditions for each step may be employed. However, it is preferred to conduct the above mentioned pyrolysis at a temperature of at least 280°C, preferably within a range of from 300 to 400°C, by vapour phase pyrolysis, whereby good results are obtainable. In this case, the retention time is not critical and is usually from 0.1 to 1,000 seconds,

preferably from 5 to 30 seconds. In general, it is desirable to shorten the retention time when the temperature is high. The material of the pyrolysis reactor is not critical and may optionally be selected from materials such as stainless steel, nickel or Hastelloy-C (trademark). The pyrolysis in the present invention is conducted preferably in the presence of a packed layer composed of fine fragments of heat resistant solid. The type of the packed layer may optionally be selected from a fixed bed type, a fluidized bed type and a moving bed type. The fine fragments of the heat resistant solid to be used for the packed layer may optionally be selected from glass beads, a metal fluoride, a metal carbonate, a metal oxide and sodium metasilicate. Further, it is preferred to carry out the reaction in a diluted condition by introducing an inert gas into the pyrolysis reaction zone. For instance, the starting acid fluoride may preliminarily be vapourized and mixed with a diluting gas and then introduced into the reactor.

As other methods for the preparation of the compound of the present invention, the following methods may be mentioned:

(1) A method in which tetrafluoroethylene and bromodifluoroacetyl fluoride are reacted in the presence of potassium fluoride and iodine to obtain 1 - bromo - 1,1,2,2,4,4,5,5 - octafluoro - 5 - iodo - 3 - oxapentane $CF_2ICF_2OCF_2CF_2Br$ and the iodine-containing ether is subjected to IF removal in a solvent with use of a metal such as zinc to convert it to the desired compound;

(2) A method in which 1,3 - diiodoperfluoropropane $ICF_2CF_2CF_2I$ is used as the starting material, the starting material is oxidized with fuming sulfuric acid to obtain perfluoroglutaroyl fluoride $FOCCF_2COF$ and 1 mole of hexafluoropropeneoxide is added thereto to obtain asymmetric diacyl fluoride

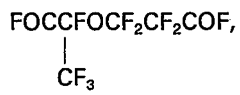

which is then thermally decomposed into fluorovinyl ether $CF_2{=}CFOCF_2CF_2COF$, which is in turn hydrolyzed into perfluoro(3-oxa-1-hexenoic acid) $CF_2{=}CFOCF_2CF_2COOH$, and the unsaturated fluorocarboxylic acid is converted to a silver salt $CF_2{=}CFOCF_2CF_2CO_2Ag$, which is then treated with bromine to obtain a saturated fluoro ether $CF_2BrCFBrOCF_2CF_2Br$, which is in turn debrominated with a metal such as zinc; and

(3) A method in which the above mentioned unsaturated fluorocarboxylic acid is treated with ammonia to form an amide, which is subjected to bromination in the presence of sodium hydroxide to obtain $CF_2BrCFBrOCF_2CF_2Br$, which is then debrominated.

The compound of the present invention is a colourless transparent liquid having a boiling point of 56°C, and can be identified by the data of $^{19}$F-NMR spectrum.

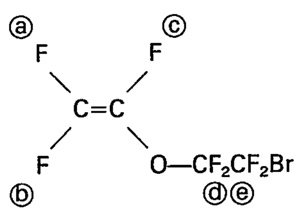

Chemical shift (ppm) coupling ($CFCl_3$ standard).

ⓐ—116 dd
ⓑ—122 tdd
ⓒ—136 tdd
ⓓ—88 m
ⓔ—70 t
d: doublet, t:triplet, m:multiplet.

Now, the present invention will be described in further detail with reference to an Example.

Example

Preparation of perfluoro(2-bromoethylvinyl ether)

72.7 g of bromodifluoroacetyl fluoride prepared by bromination of chlorotrifluoroethylene, followed by oxidation with fuming sulfuric acid, 6.25 g of cesium fluoride and 31.2 g diglyme were charged into a 200 ml stainless steel reactor and stirred at room temperature for 2 hours. While maintaining the temperature at from 4 to 6°C, 86.3 g of hexafluoropropeneoxide was introduced in 3 hours. Then, the reaction mixture was fractionated to obtain 107 g of perfluoro(5 - bromo - 3 - oxa - 2 - methyl - pentanoyl fluoride).

Into a 175 ml stainless steel reaction tube, 160 g of glass beads (GB 736 manufactured by Toshiba Glass) were packed and heated to a temperature of 320°C. Together with nitrogen gas, 101 g of the vapour of the acid fluoride obtained above was passed through the reaction tube in 2 hours, and the thermally decomposed gas was cooled, liquefied and collected. The collected liquid was fractionated to obtain 55.9 g

of perfluoro(2 - bromoethylvinyl ether) as a fraction having a boiling point of 56°C. The identification was made based on the data of $^{19}$F-NMR spectrum.

Reference Example:
Application as a comonomer capable of giving a cure site to a fluorine-containing elastomeric copolymer

Into a 1 liter stainless steel autoclave, 482 g of ion-exchanged water, 64 g of t-butanol, 6.4 g of ammonium persulfate, 12.7 g of disodium hydrogenphosphate, 3.2 g of ammonium perfluorononanate dodecahydrate and 27 g of a 5% NaOH aqueous solution were charged, and an aqueous solution prepared by dissolving 0.15 g of ferrous sulfate heptahydrate and 0.18 g of EDTA · 2Na dihydrate in 100 ml of water was further added. After cooling the autoclave with ice water, 142 g of perfluoro(2 - n - propoxypropylvinyl ether) (PHVE), and 4.8 g of perfluoro(2 - bromoethylvinyl ether) (BVE) were charged. Nitrogen was charged under pressure of up to 5 kg/cm$^2$ and then purged, and this operation was repeated three times, and thereafter, the gas was evacuated by a vacuum pump. Then, 33 g of vinylidene fluoride was fed under pressure and the autoclave was heated to a temperature of 25°C. An aqueous solution prepared by dissolving 10 g of disodium hydroxymethanesulfinate in 90 g of ion-exchanged water was gradually fed under pressure to initiate the polymerization.

The polymerization pressure was maintained at a level of from 15 to 14 kg/cm$^2$ by supplying vinylidene fluoride, and the reaction was continued for one hour. The amount of the aqueous solution of hydroxymethanesulfinic acid salt fed during this period was 42 g.

The obtained latex was freeze-coagulated and then washed and dried to obtain 153 g of a polymer. This polymer had an intrinsic viscosity of 0.72 (as measured in a solvent mixture of trifluorotrichloroethylene/dimethylformamide (90/10) at 30°C), a glass transition temperature of −35°C (as measured by DSC), and a thermal decomposition temperature of 391°C (i.e. the temperature at which a reduction of the weight has started as measured by a differential thermal balance in the air at a temperature raising rate of 10°C/min). From the elemental analysis, the composition of the copolymer was found to be VDF/PHVE/BVE in a molar ratio of 80/18/2.

To 100 parts by weight of this copolymer 1, part by weight of perhexa 2.5 B (manufactured by Nippon Oil & Fat), 4 parts by weight of triallylisocyanurate, 5 parts by weight of calcium hydroxide and 35 parts by weight of MT carbon were mixed on open rolls, subjected to press curing (170°C for 20 minutes) and then to oven curing (180°C for one hour→200°C for one hour), whereby a cured sheet (1 mm×10 cm×6 cm) was obtained. The cured rubber had a tensile strength of 100 kg/cm$^2$, an elongation of 220% and a hardness of 67 (JIS A). The tensile strength and elongation after being subjected to heat treatment at 230°C for 15 days were 70 kg/cm$^2$ and 300%, respectively. TR-10 measured by a TR tester was −30°C. The volume swell after being immersed in a fuel oil B at room temperature for 7 days was 7.0%. The volume swell after being immersed in methanol at 40°C for 3 days was 25%.

**Claim**

Perfluoro(2-bromoethylvinyl ether).

**Revendication**

Perfluoro(2-bromoéthylvinyléther).

**Patentansprüche**

Perfluor(2-bromethylvinyläther).